# EUROPEAN PATENT SPECIFICATION

(11) **EP 1 566 156 B1**
(45) Date of publication and mention of the grant of the patent: **08.10.2008**
(21) Application number: 04730633.7
(22) Date of filing: 30.04.2004
(51) Int. Cl.: A61F 7/08

(54) **HEATING ELEMENT**
HEIZELEMENT
ELEMENT DE CHAUFFAGE

(30) Priority: 11.08.2003 JP 2003291335
(43) Date of publication of application: 24.08.2005
(73) Proprietor: Mycoal Co., Ltd., Tochigi-shi, Tochigi 328-0067 (JP)
(72) Inventor: USUI, Kaoru, c/o Mycoal Co., Ltd., Tochigi-shi, Tochigi 3280067 (JP); URUME, Yukio, c/o Mycoal Co., Ltd., Tochigi-shi, Tochigi 3280067 (JP); OMAE, Hirotaka, c/o Mycoal Co., Ltd., Tochigi-shi, Tochigi 3280067 (JP)
(74) Representative: Körfer, Thomas
(86) International application number: PCT/JP2004/005836
(87) International publication number: WO 2005/013869

(56) References cited:
- EP-A- 1 557 146
- JP-A- 2002 037 728
- JP-A- 2002 177 313
- JP-A- 2003 210 509

## Description

### Technical Field

The present invention relates to a heat-generating body comprising a heat-generating portion in which a heat-generating composition causing an exothermic reaction in the presence of air is sealed in an air-permeable container in desired form, such as bag form or sheet form, and a adhesive portion formed by comprising, as a main component, a water-containing hydrophilic gel agent obtained from a hydrophilic polymeric thickening agent.

### Background Art

Ordinarily, a heat-generating body comprises a heat-generating portion formed by sealing a heat-generating composition comprising, as main components, iron powder and the like causing an exothermic reaction in the presence of air in an air-permeable container in, for example, bag form or sheet form, and a adhesive portion for attaching the heat-generating portion to skin.

A large number of heat-generating bodies in each of which a adhesive portion is constituted by a non-aqueous adhesive agent have so far been proposed.

However, since the adhesive portion constituted by the non-aqueous adhesive agent does not absorb moisture, when perspiration occurs, body fluids such as sweat and the like are retained between a surface of the skin and the adhesive portion and, then, a problem in that the heat-generating body is liable to be peeled off the skin due to reduction of a adhesive force thereof is caused.

Further, when the adhesive force of the adhesive portion is enhanced in order to allow it to be hardly peeled off the skin, there is a problem in that, when the heat-generating body is removed from the skin, skin hair and the like are pulled by the adhesive portion to cause a pain. Still further, on this occasion, there is another problem in that a corneous tissue on the surface of the skin is impaired.

Yet still further, other heat-generating bodies than the aforementioned ones in each of which the adhesive portion is constituted by a hydrophilic adhesive agent are disclosed in, for example, Patent Documents 1 and 2.

In these heat-generating bodies in each of which the adhesive portion is constituted by the hydrophilic adhesive agent, since moisture such as sweat and the like to be generated at the time of perspiration is absorbed, even when such heat-generating body as described above is removed from the skin, the skin hair is not pulled and the corneous tissue of the surface of the skin is not impaired.

However, since the adhesive portion is constituted by a water-containing hydrophilic gel comprising a hydrophilic adhesive agent ordinarily used in a wet compress or the like, even when it is contained in a non air-permeable bag, for example, at the time of transportation, the moisture in the gel is gradually transferred into the side of the heat-generating portion having a high hygroscopic property in the bag, a adhesive force of the adhesive portion of the heat-generating body is found to be reduced at the time it is taken out of the bag and, accordingly, there is a problem in that, even when it is attached to the skin, it is liable to be peeled off the skin.

Further, since the aforementioned water-containing hydrophilic gel agent gives chill feeling at the time the heat-generating body is attached to the skin or for a certain initial period after it is attached thereto, there is a problem in that, particularly in a cold time in a winter season, a user can hardly withstand the chill feeling.

Under these circumstances, it is considered that the aforementioned chill feeling is mitigated by constituting the adhesive portion of the heat-generating body such that the moisture in the water-containing hydrophilic gel agent is reduced and an amount of a moisture-retaining agent therein is increased.

However, even in the heat-generating body having such constitution as described above, since a large amount of the highly hygroscopic moisture retaining agent is still contained in the adhesive portion in a same manner as in the above case, the moisture in the heat-generating portion is transferred to the adhesive portion and, accordingly, there is a problem in that a sufficient heat-generating performance can not be obtained.
Patent Document 1: Japanese Patent No. 1874082; and
Patent Document 2: Japanese Patent No. 2744915.

### Disclosure of the Invention

Now, in order to solve the aforementioned problems, an object of the present invention is to provide a heat-generating body capable of constituting a adhesive portion comprising, as a main component, a water-containing hydrophilic gel agent obtained from a hydrophilic polymeric thickening agent and, also, being small in transition of moisture between a heat-generating portion and the adhesive portion to have an excellent heat-generating performance.

The present inventors have conducted an intensive study to attain the aforementioned object and, as a result, found that a heat-generating body, comprising a heat-generating portion formed by sealing a heat-generating composition causing an exothermic reaction in the presence of air in an air-permeable container in desired form such as bag form or sheet form and a adhesive portion formed by comprising, as a main component, a water-containing hydrophilic gel agent obtained from a hydrophilic polymeric thickening agent, which is advantageously used as a heat-generating body for being attached to skin having an excellent using property such that, by allowing a difference between critical moisture values of the aforementioned heat-generating portion and adhesive portion to be 5% or less, there is no transfer of moisture from the heat-generating composition to the water-containing hydrophilic gel agent and, accordingly, not only functions owned by the heat-generating composition such as temperature characteristics and a temperature-holding duration can effectively be performed, but also functions owned by the water-containing hydrophilic gel agent such as a adhesive force and a shape-holding property are not impaired can be obtained.

The heat-generating body according to the present invention has been achieved based on such finding as described above and is, as described in Claim 1, a heat-generating body, comprising a heat-generating portion formed by sealing a heat-generating composition causing an exothermic reaction in the presence of air in an air-permeable container and a adhesive portion formed by comprising, as a main component, a water-containing hydrophilic gel agent obtained from a hydrophilic polymeric thickening agent, being characterized in that a difference between critical moisture values of the aforementioned heat-generating portion and the adhesive portion is allowed to be 5% or less.

Further, a heat-generating body, as described in Claim 2, is the heat-generating body as described in Claim 1, being characterized in that an organic filling agent is added to the water-containing hydrophilic gel agent in the adhesive portion.

Still further, a heat-generating body, as described in Claim 3, is the heat-generating body as described in Claim 1, being characterized in that the adhesive portion is laminated on the heat-generating portion and the resultant laminate is contained in an air-tight container bag.

Yet still further, a heat-generating body, as described in Claim 4, is the heat-generating body as described in Claim 2, being characterized in that the adhesive portion is laminated on the heat-generating portion and the resultant laminate is contained in an air-tight container bag.

Even yet still further, a heat-generating body, as described in Claim 5, is the heat-generating body as described in any one of Claims 1 to 4, being characterized in that the heat-generating body is a heat-generating body for being attached to skin.

### Brief Description of the Drawings

FIG. 1 is an explanatory cross-sectional view of a sample for use in a critical moisture measurement of a heat-generating composition;
FIG. 2 is an explanatory cross-sectional view of a sample for use in a critical moisture measurement of a water-containing hydrophilic gel agent;
FIG. 3 is a graph showing a critical moisture of a heat-generating composition;
FIG. 4 is a graph showing a critical moisture of a water-containing hydrophilic gel agent;
FIG. 5 is an explanatory cross-sectional view of a heat-generating body as an embodiment according to the present invention;
FIG. 6 is an explanatory cross-sectional view of a water-containing hydrophilic gel sheet of a heat-generating body as an embodiment according to the present invention; and
FIG. 7 is an explanatory cross-sectional view of a container for a heat-generating composition in a heat-generating body as an embodiment according to the present invention.

### Best Mode for Carrying Out the Invention

Hereinafter, a best mode for carrying out the present invention will be described in detail.

In a heat-generating body according to the present invention, as described above, a difference between critical moisture values of a heat-generating portion and a adhesive potion is allowed to be 5% or less.

The term "critical moisture value" of the heat-generating portion or the adhesive portion as used herein is intended to mean a value indicating relative moisture in a state in which, at a certain relative humidity, moisture in the heat-generating portion or the adhesive portion is in an equilibrium state in which no absorption or desorption of moisture occurs and, accordingly, there is no transfer of the moisture from one portion to the other.

Ordinarily, a substance has a moisture-absorbing property and a moisture-desorbing property and, in a case of salt, for example, it has a property in which, when a relative humidity comes to be 75% or more, salt absorbs moisture in the air to be sticky (or the salt has a deliquescent property) and, accordingly, 75% of the relative humidity becomes a critical moisture value of the salt. Therefore, the salt has a property in which, in an atmosphere over the critical moisture value, it absorbs moisture (moisture absorption) while, in an atmosphere less than the critical moisture value, it desorbs moisture attached around a salt crystal into the air (moisture desorption).

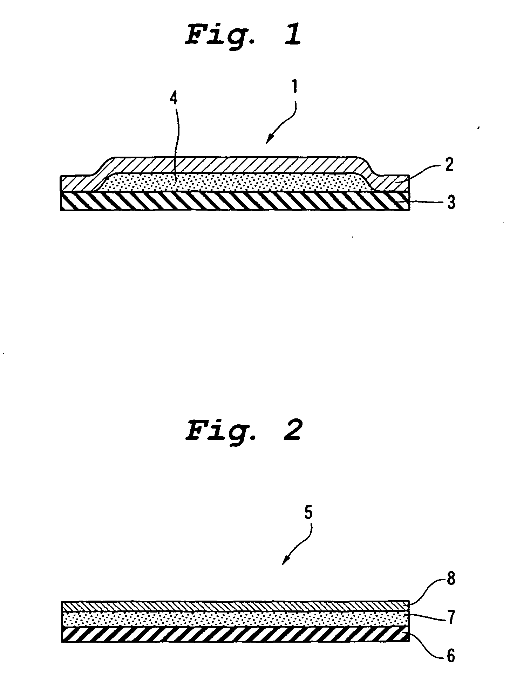

Next, a specific measuring method of the critical moisture value according to the present invention is explained with reference to FIGS. 1 to 4.

FIG. 1 is a cross-sectional view of a sample 1 of a heat-generating portion in which a heat-generating composition is contained in an air-permeable container in bag form.

The sample 1 of the heat-generating portion is produced such that an air-permeable sheet 2 and air non-permeable sheet 3 are lapped one another and, then, peripheral portions of the thus-lapped sheets are sealed so as to form an air-permeable container in bag form and, thereafter, a heat-generating composition 4 previously prepared with a desired mixture (in this case, in order to prevent an influence of a weight change to be caused by an oxidation reaction of iron, excluding iron powder having scarce moisture-absorbing/desorbing property) is loaded from an end portion of an opening of the air-permeable container and, subsequently, the end portion of the opening is heat-sealed.

FIG. 2 is a cross-sectional view of a sample 5 of a adhesive portion comprising a water-containing hydrophilic gel agent formed from a hydrophilic polymeric thickening agent.

The sample 5 of the adhesive portion is produced such that a spunlace non-woven fabric 6 lined with a polyethylene film is evenly applied with the water-containing hydrophilic gel agent 7 and, then, covered with a releasing film 8 to be in sheet form and, thereafter, the resultant sheet is punched out so as to have a same size as that of the sample 1 of the heat-generating portion.

Weights of the thus-produced samples 1 and 5 are measured to be defined as A1 and A5, respectively.

Next, each of the samples 1 and 5 are put in a constant temperature and humidity bath capable of setting a temperature at 30°C and moisture at an arbitrary relative humidity H. The samples 1 and 5 are left therein for 24 hours in a state in which the side of the air-permeable sheet 2 of the sample 1 comes to a top surface and the side of the water-containing hydrophilic gel agent 7 of the sample 5 comes to a top side surface. Then, immediately after the samples 1 and 5 are taken out of the constant temperature and humidity bath, weights of the samples 1 and 5 are measured to be defined as B1 and B5, respectively. In this case, B1 and B5 are measured at a relative humidity H in the range of from 30% to 100% while changing the relative humidity H by 10% in an incremental manner.

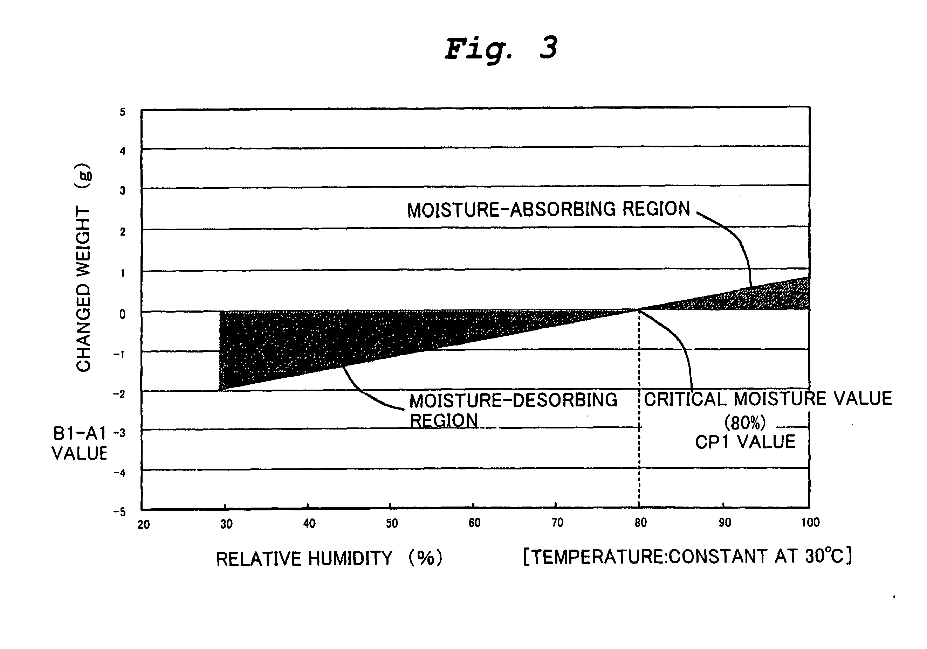

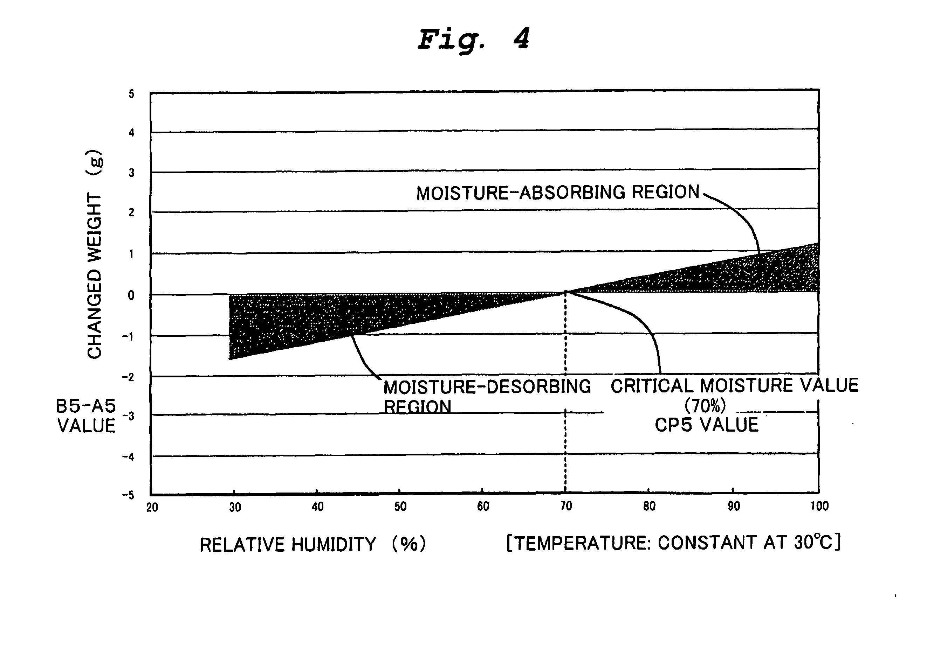

Thereafter, the relative humidity H (%) at the temperature of 30°C is plotted in abscissa and a weight change (B1-A1) at the relative humidity H (%) is plotted in ordinate, to thereby complete a graph as shown in FIG. 3. In a same manner as in the graph as shown in FIG. 3, the relative humidity H (%) at the temperature of 30°C is plotted in abscissa and a weight change (B5-A5) at the relative humidity H (%) is plotted in ordinate, to thereby complete a graph as shown in FIG. 4.

In FIG. 3, when the weight change (B1-A1) is in a negative value, the sample 1 loses weight due to desorbing moisture outside, while, when the weight change (B1-A1) is in a positive value, the sample 1 increases on weight due to absorbing moisture from outside.

Now, a point at which there is no weight change (B1-A1=0) in the sample 1, namely, a relative humidity in which there is no absorption or desorption of moisture comes to be a critical moisture value CP1 (%) of the sample 1. Same can be said with the sample 5. In FIG. 4, a point at which there is no weight change (B5-A5=0) in the sample 5, namely, a relative humidity in which there is no absorption or desorption of moisture comes to be a critical moisture value CP5 (%) of the sample 5.

As described above, the critical moisture values of the heat-generating portion and the adhesive portion in the heat-generating body can be measured as CP1 and CP5, respectively.

A difference between the critical moisture values of the heat-generating portion and the adhesive portion is indicated as an absolute value of (CP1-CP5). It is necessary to allow the value to be 5% or less and, preferably, 2% or less. When it is over 5%, the transfer of the moisture between the heat-generating portion and the adhesive portion becomes unduly large and, then, functions owned by the heat-generating composition in the heat-generating portion and the water-containing hydrophilic gel agent in the adhesive portion are deteriorated and, accordingly, the heat generating body becomes unfavorable to be used as that of an attaching type.

Further, as for the critical moisture value of the heat generating portion, a desired critical moisture value can be obtained by adjusting a mixing ratio of a moisture-absorbing material and/or a moisture-desorbing material in the heat-generating composition, selecting a material of an air-permeable container or the like. Also, as for the critical moisture value of the adhesive portion, a desired critical moisture value can be obtained by adjusting a mixing ratio of a moisture-absorbing material and/or a moisture-desorbing material in the adhesive composition. Any adjusting method of the critical moisture value is permissible without any particular restriction.

Still further, the heat-generating composition which constitutes the heat-generating portion is not particularly limited, so long as it has components of a known heat-generating composition as ordinarily called as a chemical body warmer. As an example of the composition, it comprises, as main components, metallic powder such as iron powder and water, and, further, an inorganic chloride, activated carbon which has functions of causing an oxidation reaction of the metallic powder, adjusting a pH value and performing a catalytic action, or a water-retaining agent which prevents an entire heat-generating composition from becoming sticky due to water and the like.

The inorganic chloride, which has functions of breaking an oxide film on a surface of the metallic powder of the heat-generating composition to attain continuous progress of the oxidation reaction, is mixed at a ratio of from 2 parts by mass to 10 parts by mass based on 100 parts by mass of the heat-generating composition. Further, according to the present invention, since a ratio of the inorganic chloride in the heat-generating composition is related with the critical moisture value of the heat-generating composition, the critical moisture value of the heat-generating composition can be adjusted by adjusting the inorganic chloride in the heat-generating composition.

As for such inorganic chlorides, for example, sodium chloride, potassium chloride, magnesium chloride, calcium chloride, potassium sulfate, magnesium sulfate and sodium sulfate can be used. Among these compounds, from the reason that an adjustment of the critical moisture value can particularly easily be performed, it is preferable to choose sodium chloride, magnesium chloride and calcium chloride which have a deliquescent property and, then, add any one of them to the heat-generating composition.

Further, as for metallic powder, cast iron powder, reduced iron powder, electrolytic iron powder and the like can be used.

As for the activated carbon, charcoal prepared from husks of coconuts, wood and peat can be used.

As for such water-retaining agents, vermiculite, perlite, cristobalite, silica gel, wood powder, a water-absorbing polymer and the like can be used.

The heat-generating composition is contained in an air-permeable container. The air-permeable container is constituted by, for example, an air-permeable packaging material in flat form and an air non-permeable packaging material in sheet form. The air-permeable packaging material in flat form is constituted by a base material and a covering material and they are not particularly limited, so long as at least one of them has air permeability; even when a water-containing hydrophilic gel agent layer is provided thereto, the heat-generating composition generates heat, to thereby obtain a desired temperature; and, when the heat-generating composition is contained therein, the heat-generating composition thus contained therein is not leaked.

Next, a water-containing hydrophilic gel agent formed from a hydrophilic polymeric thickening agent to be used in the present invention will be described.

From the standpoint of an excellent adhesion to skin and an excellent shape-retaining property, the water-containing hydrophilic gel agent is constituted by adding a curing agent, water, a moisture-retaining agent and the like to a hydrophilic polymeric thickening agent such as polyacrylic acid, a salt of polyacrylic acid or a cellulose derivative.

Water is not particularly limited in purity, kind and the like.

Further, as for such moisture-retaining agents, mentioned are polyhydric alcohols and saccharides. Specific examples of the polyhydric alcohols include ethylene glycol, diethylene glycol, triethylene glycol, propylene-ethylene glycol, dipropylene glycol, butylene glycol, hexylene glycol, glycerin, diglycerin, polyethylene glycol, polypropylene glycol and polyglycerin. Specific examples of the saccharides include xylitol, sorbitol, maltitol and starch sugar.

Since a mixing ratio between the moisture-retaining agent and water is related with a critical moisture value of the water-containing hydrophilic gel agent according to the present invention, the critical moisture value of the water-containing hydrophilic gel agent can also be adjusted by adjusting the mixing ratio between the moisture-retaining agent and water.

Further, the moisture-retaining agent is ordinarily mixed at the rate of 60 parts by mass or less based on 100 parts by mass of the water-containing hydrophilic gel agent. In a case in which the mixing ratio of the moisture-retaining agent is over 60 parts by mass, when the heat-generating body is attached to skin, it is liable to be peeled off the skin. Still further, it is preferable that the moisture-retaining agent is mixed in the range, based on 100 parts by mass of the water-containing hydrophilic gel agent, of from 40 parts by mass to 60 parts by mass. The reason is that, when the moisture-retaining agent is less than 40 parts by mass, a water content becomes large and, accordingly, a chill feeling becomes large in intensity.

Further, it is preferable that an organic filling agent is allowed to be contained in the water-containing gel agent in the adhesive portion. An amount of the organic filling agent to be added is preferably in the range, based on 100 parts by mass of the water-containing hydrophilic gel agent, of approximately from 1 part by mass to 30 parts by mass. The reason is that the chill feeling to be given at the time the heat-generating body is attached to skin is further suppressed.

Examples of such organic filling agents include crystalline cellulose, wood powder, dry vegetable powder, pulp, regenerated cellulose and fiber chips.

A molecular weight and a structure, for example, a linear or branched form of the aforementioned polyacrylic acid are not particularly limited.

Further, specific examples of the salts of polyacrylic acid include sodium polyacrylate, potassium polyacrylate, polyacrylic acid monoethanolamine, polyacrylic acid diethanolamine, polyacrylic acid triethanolamine and a polyacrylic acid ammonium salt.

Still further, specific examples of the cellulose derivatives include carboxymethyl cellulose, sodium carboxymethyl cellulose, hydroxymethyl cellulose, hydroxyethyl cellulose, hydroxypropyl cellulose, hydroxypropyl methyl cellulose, hydroxypropyl ethyl cellulose, methyl cellulose, ethyl cellulose, ethyl hydroxymethyl cellulose and cationated cellulose.

Yet still further, by adding any one of such water-soluble polymers as well as polymeric thickening agents as exemplified as sodium alginate, propylene glycol alginate ester, sodium starch glycolate, sodium starch phosphate ester, polyvinyl alcohol, polyvinyl pyrrolidone, polyvinyl pyrrolidone polyethylene oxide, polyvinyl methyl ether, a cerboxyvinyl polymer, polyacrylamide, polyethylene glycol, polyethylene oxide, guar gum, gum Arabic, tragacanth gum, gum karaya, carrageenan, agar, xanthan gum, gellan gum, curdlan, pullulan, pectin, dextrin, chitin, chitosan, chitosamine and gelatin, adhesion to skin can further be enhanced and, also, a adhesive force of the water-containing hydrophilic gel agent can be enhanced.

Even yet still further, the curing agent causes cross-linking with polyacrylic acid, a polyacrylic acid salt, a cellulose derivative or the like. Specific examples of divalent metallic compounds of such curing agents include calcium hydroxide, calcium carbonate, calcium sulfate, calcium nitrate, calcium chloride, calcium acetate, calcium oxide, calcium phosphate, magnesium hydroxide, magnesium carbonate, magnesium sulfate, magnesium nitrate, magnesium chloride, magnesium silicate, magnesium oxide, magnesium alumina hydroxide, magnesium aluminometasilicate, magnesium alminosilicate and synthetic hydrotalcite. Specific examples of trivalent metallic compounds thereof include potassium alum, ammonium alum, ferric alum, aluminum hydroxide, aluminum sulfate, aluminum chloride, aluminum aluminoglycinatoacetate, aluminum oxide, silicic acid-containing aluminum and aluminum metasilicate.

Furthermore, in the water-containing hydrophilic gel agent, other components than those described above which are ordinarily used in a wet compress, an application agent, a cosmetic gel sheet and the like may appropriately be mixed, so long as the effect of the present invention is not impaired. Examples of such components include inorganic powder, a percutaneous absorption medical agent, an antioxidant, an antiseptic agent, a perfume, a coloring material, a coloring pigment, an emulsifier, an antiallergic agent, a cosmetic component and the like.

Still furthermore, it is preferable that the adhesive portion is laminated to the heat-generating portion and the resultant laminate is contained in an air-tight container bag. Transfer of moisture from the adhesive portion to the heat-generating portion does not occur in the air-tight container bag during a period of from the time the heat-generating body is produced till the time it is used and, accordingly, the heat-generating performance as the heat-generating body or a adhesive property of the adhesive portion is not deteriorated.

Yet still furthermore, as for materials to constitute the air-tight container bag, they are not limited to those having a perfect air-tightness and, specifically, a material made of aluminum, PET or the like can constitute the air-tight container bag.

### EXAMPLES

The present invention will be specifically described below with reference to Examples and Comparative Examples; however, the present invention is not limited thereto.

Before preparing a heat-generating body for being attached to skin of Examples and Comparative Examples, a sample of each of a heat-generating portion and a adhesive portion was prepared.

Firstly, components as shown in Table 1 were mixed and stirred in an ordinary manner, to thereby prepare a heat-generating composition.

Critical moisture values of heat-generating compositions of mixtures 1 to 3 were measured in a manner as described above with reference to FIGS. 1 and 3 to be 82%, 90% and 95%, respectively. Further, as an air-permeable sheet 2 for use in measurement, used was a porous film (trade name: BREATHRON; available from Nitto Lifetec Corporation) formed by laminating a fine porous polyethylene sheet on a nylon spunbonded non-woven fabric, while, as an air non-permeable sheet 3, used was such sheet as formed by laminating a polyethylene film on a nylon spunbonded non-woven fabric (available from Asahi Kasei Corporation).

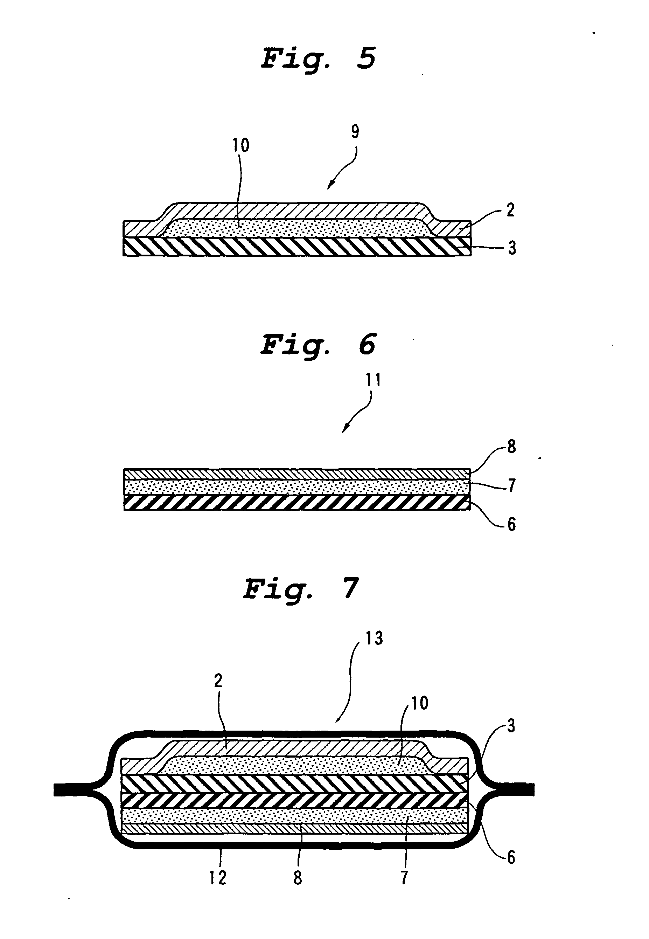

Next, as shown in FIG. 5, about 10 g each of heat-generating compositions 10 of mixtures 1 to 3 was measured and filled in an air-permeable container 9 which has been prepared such that the air-permeable sheet 2 comprising the porous film (trade name: BREATHRON; available from Nitto Lifetec Corporation) formed by laminating a fine porous polyethylene sheet on a nylon spunbonded non-woven fabric and the air non-permeable sheet 3 formed by laminating a polyethylene film on a nylon spunbonded non-woven fabric (available from Asahi Kasei Corporation) were punched out to be in a rectangular shape having sizes of 7 cmx10 cm and, then, lapped one on the other and, thereafter, peripheral portions thereof were sealed with each other to form an air-permeable container 9 in bag form and, then, each heat-generating composition 10 was filled therein and, thereafter, an opening of the air-permeable container 9 was sealed, to thereby prepare a heat-generating body.

**Table 1**

| Component | | Mixture 1 | Mixture 2 | Mixture 3 |
|---|---|---|---|---|
| Component of heat-generat ing composition (mass%) | Iron powder | 52 | 56 | 58 |
| | Activated carbon | 5 | 5 | 5 |
| | Water-retaining agent | 5 | 5 | 5 |
| | Water | 30 | 30 | 30 |
| | Sodium chloride | 8 | 4 | 2 |
| Total | | 100 | 100 | 100 |
| Critical moisture value (%) | | 82 | 90 | 95 |

Next, components as shown in Table 2 were mixed and stirred by a mixer in an ordinary manner, to thereby prepare a water-containing hydrophilic gel agent. Critical moisture values of such water-containing hydrophilic gel agents of mixtures 4 to 6 were measured in a manner as described above with reference to FIGS. 2 and 4 to be 96%, 91% and 80%, respectively. Further, as a spunlace non-woven fabric 6 lined with a polyethylene film, a spunlace non-woven fabric (basis weight : 55 g/m²) of rayon fibers lined with a polyethylene film was used.

Next, as shown in FIG. 6, from 1000 to 1200 g/m² of the water-containing hydrophilic gel agent 7 each of mixtures 4 to 6 was uniformly applied on a spunlace non-woven fabric 6 (basis weight: 55 g/m²) of rayon fabrics lined with a polyethylene film and, then, a releasing film 8 of polyethylene terephthalate which has been subjected to an embossing treatment was overlaid on the resultant spunlace non-woven fabric 6 and, thereafter, the resultant assembly was molded to be in sheet form, punched out so as to have a same size as that of the container 9 in which the heat-generating composition was filled, to thereby prepare a adhesive portion 11 comprising the water-containing hydrophilic gel agent.

**Table 2**

| Component | | | Mixture 4 | Mixture 5 | Mixture 6 |
|---|---|---|---|---|---|
| Adhesive portion composition (mass%) | Polyacrylic acid | Linear polyacrylic acid | 3.00 | 3.00 | 3.00 |
| | Polyacrylic acid salt | Sodium polyacrylate | 5.00 | 5.00 | 5.00 |
| | Cellulose derivative | Carboxymethyl cellulose | 2.00 | 2.00 | 2.00 |
| | Moisture-retaining agent | Glycerin | 20.00 | 30.00 | 40.00 |
| | Polyvalent metallic compound | Magnesium aluminometasilicate | 0.20 | 0.20 | 0.20 |
| | Organic filling agent | Crystalline cellulose | 2.00 | 2.00 | 2.00 |
| | Others | Sodium edetate | 0.05 | 0.05 | 0.05 |
| | | Tartaric acid | 0.05 | 0.05 | 0.05 |
| | Purified water | | 67.70 | 57.70 | 47.70 |
| Total | | | 100.00 | 100.00 | 100.00 |
| Critical moisture value (%) | | | 96 | 91 | 80 |

### Example 1

After weights of a heat-generating portion 9 constituted by a heat-generating composition (critical moisture value: 82%) of mixture 1 and a adhesive portion 11 constituted by a water-containing hydrophilic gel agent (critical moisture value: 80%) of mixture 6 were measured, as shown in FIG. 7, an air non-permeable sheet 3 of the heat-generating portion 9 and a spunlace non-woven fabric 6 of rayon fabrics lined with a polyethylene film of the adhesive portion 11 were lapped one on the other, to thereby prepare a heat-generating body 13 which is, then, sealed in an air-tight container bag 12.

### Example 2

A heat-generating portion 9 was constituted by a heat-generating composition (critical moisture value: 90%) of mixture 2 and a adhesive portion 11 was constituted by a water-containing hydrophilic gel agent (critical moisture value: 91%) of mixture 5. After weights of the heat-generating portion 9 and the adhesive portion 11 were measured, the resultant heat-generating body 13 was sealed in an air-tight container bag 12 in a same manner as in Example 1.

### Example 3

A heat-generating portion 9 was constituted by a heat-generating composition (critical moisture value: 95%) of mixture 3 and a adhesive portion 11 was constituted by a water-containing hydrophilic gel agent (critical moisture value: 96%) of mixture 5. After weights of the heat-generating portion 9 and the adhesive portion 11 were measured, the resultant heat-generating body 13 was sealed in an air-tight container bag 12 in a same manner as in Example 1.

### Comparative Example 1

A heat-generating portion 9 was constituted by a heat-generating composition (critical moisture value: 82%) of mixture 1 and a adhesive portion 11 was constituted by a water-containing hydrophilic gel agent (critical moisture value: 91%) of mixture 5. After weights of the heat-generating portion 9 and the adhesive portion 11 were measured, the resultant heat-generating body 13 was sealed in an air-tight container bag 12 in a same manner as in Example 1.

### Comparative Example 2

A heat-generating portion 9 was constituted by a heat-generating composition (critical moisture value: 90%) of mixture 2 and a adhesive portion 11 was constituted by a water-containing hydrophilic gel agent (critical moisture value: 80%) of mixture 6. After weights of the heat-generating portion 9 and the adhesive portion 11 were measured, the resultant heat-generating body 13 was sealed in an air-tight container bag 12 in a same manner as in Example 1.

### Comparative Example 3

A heat-generating portion 9 was constituted by a heat-generating composition (critical moisture value: 90%) of mixture 2 and a adhesive portion 11 was constituted by a water-containing hydrophilic gel agent (critical moisture value: 96%) of mixture 4. After weights of the heat-generating portion 9 and the adhesive portion 11 were measured, the resultant heat-generating body 13 was sealed in an air-tight container bag 12 in a same manner as in Example 1.

After the heat-generating body 13 sealed in the air-tight container bag 12 of each of Examples 1 to 3 and Comparative Examples 1 to 3 was left for 30 days in a constant temperature and moisture bath set at a temperature of 40°C and at a moisture of 50%, the heat-generating body 13 was taken out of the air-tight container bag 12 and, then, the weights of the heat-generating portion 9 and the adhesive portion 11 were measured, to thereby obtain volumes of changes of moisture. The results are shown in Table 3.

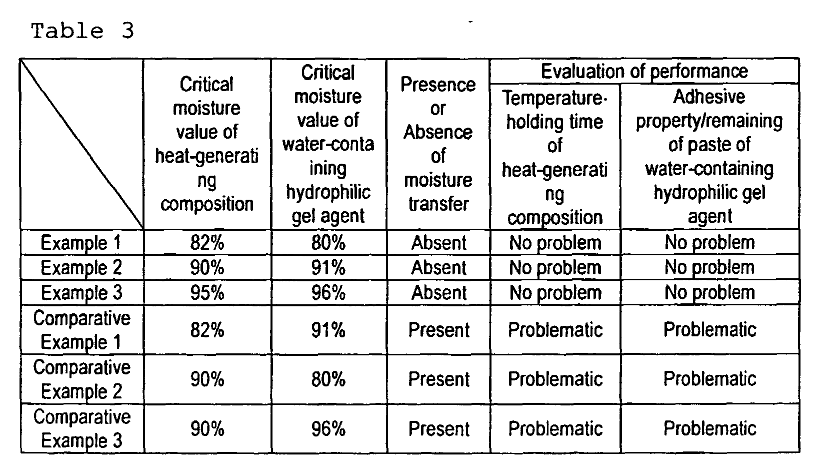

Thereafter, an evaluation of performance of heat-generating characteristics (initial temperature rising property and temperature holding time) of the heat-generating portion was conducted and, also, an evaluation of performance of a adhesive property of the adhesive portion was conducted. The results are shown in Table 4.

Further, in the evaluation of the heat-generating portion, with reference to the initial temperature rising property, when a period between the time heat started to be generated and the time a temperature reached 40°C or more was short, the heat-generating portion was evaluated as "favorable", while, when it was relatively long, the heat-generating portion was evaluated as "unfavorable". Still further, with reference to the temperature holding time, when a temperature of 40°C or more was held for 6 hours or more, the heat-generating portion was evaluated as "no problem", while, when it was not, the heat-generating portion was evaluated as "problematic".

Yet still further, as far as an evaluation of the adhesive portion is concerned, presence or absence of peeling-off at the time 6 hours have passed after the heat-generating body was attached to skin of a flat portion of a back of a human body and presence or absence of the adhesive portion remaining on skin after the heat-generating body was removed from the skin were observed.

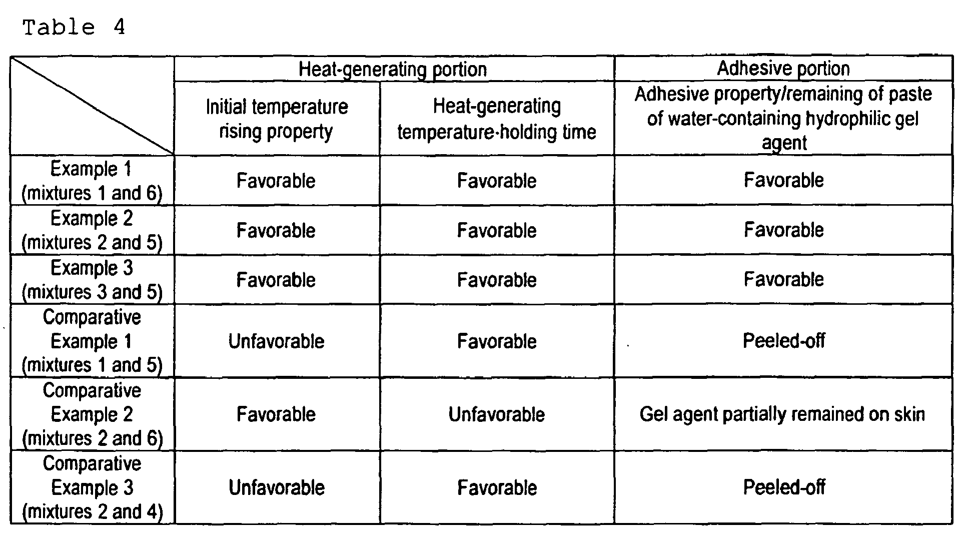

As is apparent from the results shown in Table 4, in Examples 1 to 3, there is no transfer of moisture and heat-generating bodies favorable in the heat-generating characteristics and the adhesive property were obtained. On the other hand, in the heat-generating bodies in Comparative Examples 1 to 3, it was observed that there was a transfer of moisture and they were problematic in the heat-generating characteristics and the adhesive property.

### Industrial Applicability

According to the present invention, since transfer of moisture (substance necessary for performing an oxidation reaction with iron) contained in a heat-generating composition into a water-containing hydrophilic gel agent which primarily constitutes a adhesive portion can be suppressed, an initial temperature rising property at the time of heat-generation or heat-generating characteristics such as a maximum temperature and an optimum temperature holding time become excellent.

Further, since moisture in the water-containing hydrophilic gel agent in the adhesive portion which contributes to a gel formation or a adhesive force is not transferred into the heat-generating composition in a heat-generating portion, a shape-holding property of the adhesive portion, cohesion or a feeling of attachment to skin can be maintained without being impaired.

Still further, according to the present invention, since a ratio of a moisture-retaining agent in the adhesive portion is high, a water content in the adhesive portion can be reduced and, accordingly, a chill feeling at the time the heat-generating body is attached to skin or for a certain initial period of time after the heat-generating body is attached to skin, which is given by a conventional heat-generating body, can be reduced.

Yet still further, by allowing an organic filling agent to be contained in the adhesive portion, the chill feeling can further be reduced.

Furthermore, by sealing the heat-generating body in an air-tight container bag, heat-generating characteristics or the adhesive performance of the heat-generating body is not deteriorated during a period between the time the heat-generating body is produced and the time it is used by a consumer.

## Claims

1. A heat-generating body (13), comprising a heat-generating portion (9) formed by sealing a heat-generating composition (10) causing an exothermic reaction in the presence of air in an air-permeable container (2, 3) in desired form such as bag form or sheet form and a adhesive portion (11) formed by comprising, as a main component, a water-containing hydrophilic gel agent (7) obtained from a hydrophilic polymeric thickening agent, being **characterized in that** a difference between critical moisture values of the heat-generating portion and the adhesive portion is 5% or less, wherein the critical moisture value is a value indicating relative moisture in a state in which, at a certain relative humidity, moisture in the heat-generating portion or the adhesive portion is in an equilibrium state in which no absorption or desorption of moisture occurs.

2. The heat-generating body as set forth in Claim 1, being **characterized in that** an organic filling agent is added in the water-containing hydrophilic gel agent in the adhesive portion.

3. The heat-generating body as set forth in Claim 1, being **characterized in that** the adhesive portion is laminated on the heat-generating portion and the resultant laminate is contained in an air-tight container bag (12).

4. The heat-generating body as set forth in Claim 2, being **characterized in that** the adhesive portion is laminated on the heat-generating portion and the resultant laminate is contained in an air-tight container bag.

5. The heat-generating body as set forth in any one of Claims 1 to 4, being **characterized in that** the heat-generating body is a heat-generating body for being attached to skin.

## Patentansprüche

1. Wärmeerzeugender Körper (13), welcher einen wärmeerzeugenden Abschnitt (9), welcher durch Abdichten einer wärmeerzeugenden Zusammensetzung (10) gebildet ist, welche eine exotherme Reaktion in der Anwesenheit von Luft in einem luftdurchlässigen Behälter (2, 3) einer gewünschten Form, wie beispielsweise eine Beutelform oder eine Plattenform, verursacht, sowie einen Klebeabschnitt (11) aufweist, welcher durch Umfassen, als eine Hauptkomponente, eines wasserenthaltenden hydrophilen Gelmittels (7) gebildet ist, welches aus einem hydrophilen polymeren Verdickungsmittel (7) erhalten wird, **dadurch gekennzeichnet, dass** ein Unterschied zwischen kritischen Feuchtigkeitswerten des wärmeerzeugenden Abschnitts und des Klebeabschnitts 5 % oder weniger ist, wobei der kritische Feuchtigkeitswert ein Wert ist, welcher eine relative Feuchtigkeit in einem Zustand anzeigt, in welchem bei einer bestimmten relativen Luftfeuchtigkeit Feuchtigkeit in dem wärmeerzeugenden Abschnitt oder dem Klebeabschnitt in einem Gleichgewichtszustand ist, in welchem keine Absorption oder Desorption von Feuchtigkeit auftritt.

2. Wärmeerzeugender Körper gemäß Anspruch 1, **dadurch gekennzeichnet, dass** ein organisches Füllmittel dem wasserenthaltenden hydrophilen Gelmittel in dem Klebeabschnitt hinzugefügt ist.

3. Wärmeerzeugender Körper gemäß Anspruch 1, **dadurch gekennzeichnet, dass** der Klebeabschnitt auf den wärmeerzeugenden Abschnitt auflaminiert ist und das resultierende Laminat in einem luftdichten Behälterbeutel (12) enthalten ist.

4. Wärmeerzeugender Körper gemäß Anspruch 2, **dadurch gekennzeichnet, dass** der Klebeabschnitt auf den wärmeerzeugenden Abschnitt auflaminiert ist und das resultierende Laminat in einem luftdichten Behälterbeutel enthalten ist.

5. Wärmeerzeugender Körper gemäß irgendeinem der Ansprüche 1 bis 4, **dadurch gekennzeichnet, dass** der wärmeerzeugende Körper ein wärmeerzeugender Körper zum Anbringen an Haut ist.

## Revendications

1. Un corps thermogène (13), comprenant une partie thermogène (9) formée en scellant une composition thermogène (10) entraînant une réaction exothermique en présence d'air dans un récipient perméable à l'air (23) sous une forme souhaitée telle que la forme d'un sac ou la forme d'une feuille et une partie adhésive (11) formée en comprenant, en tant que composant principal, un agent de type gel hydrophile contenant de l'eau (7) obtenu à partir d'un agent épaississant polymère hydrophile, **caractérisé en ce que** la différence entre les valeurs d'humidité critique de la partie thermogène et la partie adhésive est inférieure ou égale à 5 %, la valeur d'humidité critique étant une valeur indiquant l'humidité relative dans un état dans lequel, à une certaine humidité relative, l'humidité dans la partie thermogène ou la partie adhésive est dans un état d'équilibre dans lequel il n'y a aucune absorption ni désorption de l'humidité.

2. Corps thermogène tel que déterminé selon la revendication 1, **caractérisé en ce qu'**un agent de charge organique est ajouté dans l'agent de type gel hydrophile contenant de l'eau dans la partie adhésive.

3. Corps thermogène tel que déterminé selon la revendication 1, **caractérisé en ce que** la partie adhésive est stratifiée sur la partie thermogène et le stratifié résultant est contenu dans un sac de type récipient hermétique (12).

4. Corps thermogène tel que déterminé selon la revendication 2, **caractérisé en ce que** la partie adhésive est stratifiée sur la partie thermogène et le stratifié résultant est contenu dans un sac de type récipient hermétique.

5. Corps thermogène tel que déterminé selon l'une quelconque des revendications 1 à 4, **caractérisé en ce que** le corps thermogène est un corps thermogène devant être attaché à la peau.
